# EUROPEAN PATENT SPECIFICATION

(11) **EP 3 030 324 B1**
(45) Date of publication and mention of the grant of the patent: **11.03.2020**
(21) Application number: 13753519.1
(22) Date of filing: 01.08.2013
(51) Int. Cl.: A61Q 5/12, A61K 8/898, A61K 8/73, A61K 8/81, A61K 8/34

(54) **COMPOSITION COMPRISING AN AMINOSILICONE, A CATIONIC SURFACTANT, AN ASSOCIATIVE POLYMER AND A THICKENING AGENT**
ZUSAMMENSETZUNG MIT EINEM AMINOSILIKON, EINEM KATIONISCHEN TENSID, EINEM ASSOZIATIVEN POLYMER UND EINEM VERDICKUNGSMITTEL
COMPOSITION COMPORTANT UNE SILICONE AMINÉE, UN TENSIOACTIF CATIONIQUE, UN POLYMÈRE ASSOCIATIF ET UN AGENT ÉPAISSISSANT

(43) Date of publication of application: 15.06.2016
(73) Proprietor: L'OREAL, 75008 Paris (FR); Fernandes, Pedro Aprigliano, 21930-220 Rio de Janeiro (BR)
(72) Inventor: FERNANDES, Pedro Aprigliano, 21930-220 Rio de Janeiro (BR); PINHO, Fabiana, 26255-230 Rio de Janeiro (BR)
(74) Representative: Casalonga
(86) International application number: PCT/BR2013/000297
(87) International publication number: WO 2015/013779

(56) References cited:
- JP-A- 2006 273 732
- US-A1- 2010 034 765
- US-A1- 2011 200 548
- US-A1- 2013 164 243
- DATABASE GNPD [Online] MINTEL; 1 May 2013 (2013-05-01), "Conditioner", XP002723723, Database accession no. 2065228
- DATABASE GNPD [Online] MINTEL; 1 July 2013 (2013-07-01), "Conditioner", XP002723724, Database accession no. 2050595
- "https://www.momentive.com/workarea/downlo adasset.aspx?id=22998", , 24 February 2011 (2011-02-24), XP002723725, Retrieved from the Internet: URL:https://www.momentive.com/workarea/dow nloadasset.aspx?id=22998 [retrieved on 2014-04-24]
- ROSALÍA RODRÍGUEZ ET AL: "Rheological Evaluation of the Interactions between Cationic Celluloses and Carbopol 974P in Water", BIOMACROMOLECULES, vol. 2, no. 3, 1 September 2001 (2001-09-01), pages 886-893, XP055393914, ISSN: 1525-7797, DOI: 10.1021/bm010049c

## Description

The present invention relates to a composition for treating keratin fibres, in particular human keratin fibres such as the hair, comprising (i) one or more amino silicones, (ii) one or more cationic surfactants different from (i), (iii) one or more associative polymers and (iv) one or more thickening agents different from (iii).

The invention also concerns a process for treating keratin fibres, in particular human keratin fibres such as the hair, and a use for hair care employing the said composition.

Many people are unsatisfied with the way their hair looks, and have difficulty in styling it. Hair is generally damaged and embrittled by the action of external atmospheric agents such as light and bad weather, and also by mechanical or chemical treatments, such as brushing, combing, dyeing, bleaching, permanent-waving and/or relaxing.

Hair is thus damaged by these various factors and may over time become dry, coarse or dull, especially in fragile areas, and more particularly at the ends leading to the apparition of split ends.

Thus, to overcome these drawbacks, it is common practice to resort to hair care products using compositions that condition the hair appropriately, giving it satisfactory cosmetic properties, especially in terms of smoothness, sheen, softness, suppleness, lightness, a natural feel and good disentangling properties, while at the same time being capable of minimizing the split ends.

These hair care compositions may be, for example, conditioning shampoos, hair conditioners, masks or sera, and may be in the form of gels, hair lotions or care creams that are more or less thick.

These compositions may comprise silicones, which are used as conditioning agents, so as to give the hair a satisfactory level of care, especially in terms of softness, smoothness and suppleness, and to manage the reduction of split ends.

For example, Mintel Conditioner (2065228) is a commercial product which employs bis(C13-C15 alkoxy) PG amodimethicone (amino functional siloxane glycol polymer (i) of present claim 1). This formulation comprises also a non-associative polymer (*i.e.* the quaternized hydroxyethyl cellulose polyquaternium-10).

However, silicones often have the drawback of making the hair very lank and heavy, which leads to the phenomenon commonly known as the "build-up effect". In other words, silicones become deposited in a large amount of material on the hair, which has the consequence of making the head of hair lank and of limiting the use of silicones for conditioning the hair.

These hair care products may also the drawback of giving to the hair an unnatural aspect.

Therefore, there is a real need to develop compositions, for example hair conditioners, that do not have the combination of drawbacks described above, i.e. which are capable of treating and positively nourishing the hair, especially sensitized hair, namely by affording it softness, smoothness and suppleness while at the same time leaving it more light and natural.

The Applicant has discovered, surprisingly, that it is possible to formulate compositions for the treatment of keratin fibres, which have the desired properties, by combining in these compositions one or more amino silicones, one or more cationic surfactants, one or more associative polymers and one or more thickening agents different from the associative polymers previously mentioned.

In particular, it has been found that such a combination allows depositing selectively a suitable amount of amino silicones on hair, especially on sensitized hair, in order to improve the cosmetic properties conferred to it while at the same time leaving it more light and natural. In other words, the treated hair is less lank and heavy while the cosmetic properties are improved.

More particularly, the deposits of silicones lead to softer, smoother and suppler hair and the number of splits ends are also reduced.

The present invention relates especially to a composition comprising:
(i) one or more amino silicones,
(ii) one or more cationic surfactants different from (i),
(iii) one or more associative polymers, and
(iv) one or more thickening agents different from the associative polymers (iii),
the associative polymers being cationic amphiphilic associative polymers chosen from quaternized hydroxyethylcelluloses modified with at least one alkyl group containing at least 8 carbon atoms,
the associative polymers being present in said composition in an amount of from 0.01 wt% to 5 wt%, relative to the total weight of the composition.

The composition of the present invention is able to improve the cosmetic properties of keratin fibres, in particular human keratin fibres such as the hair, especially in terms of softness, smoothness and suppleness while at same time giving the hair a more natural look and feeling.

The present invention also relates to a process for treating the hair, comprising the application to the said fibres of the composition according to the invention.

The invention also relates to the use of the composition according to the invention as a hair conditioner, preferably used after the application of a shampoo.

Other subjects and characteristics, aspects and advantages of the invention will emerge even more clearly on reading the description and the example that follows.

### Amino silicone

The term "amino silicone" according to the present invention means any polyaminosiloxane, i.e. any polysiloxane comprising at least one primary, secondary or tertiary amine function or a quaternary ammonium group.

Preferably, the amino silicone(s) used in the composition according to the present invention are chosen from:
(a) the compounds corresponding to formula (I) below:

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)

   in which:
   T is a hydrogen atom or a phenyl, hydroxyl (-OH) or C₁-C₈ alkyl radical, and preferably methyl, or a C₁-C₈ alkoxy, preferably methoxy,
   a denotes the number 0 or an integer from 1 to 3, and preferably 0,
   b denotes 0 or 1, and in particular 1,
   m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10;
   R¹ is a monovalent radical of formula -C_{q}H_{2q}L in which q is a number from 2 to 8 and L is an optionally quaternized amino group chosen from the following groups:
      - N(R²)-CH₂-CH₂-N(R²)₂;
      - N(R²)₂; -N⁺(R²)₃Q⁻ such as -N⁺(R²)(H)₂Q⁻, -N⁺(R²)₂HQ⁻,
      - N(R²)-CH₂-CH₂-N⁺(R²)(H)₂Q⁻,
   in which R² denotes a hydrogen atom, a phenyl, a benzyl or a saturated monovalent hydrocarbon-based radical, for example a C₁-C₂₀ alkyl radical, and Q⁻ represents a halide ion, for instance fluoride, chloride, bromide or iodide.

In a particular embodiment, the amino silicones corresponding to the definition of formula (I) are chosen from the compounds corresponding to formula (IA) below: 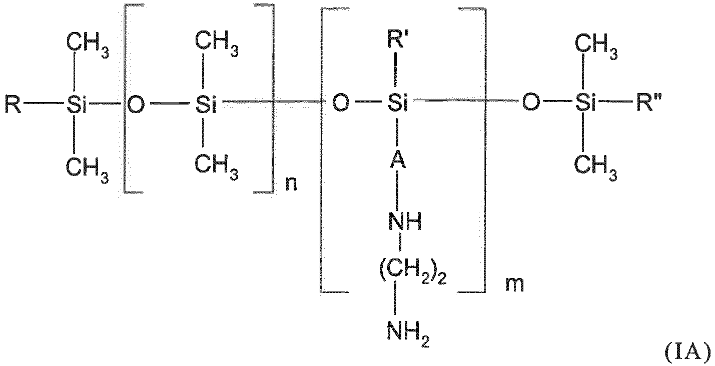 in which R, R' and R", which may be identical or different, denote a C₁-C₄ alkyl radical, preferably CH₃; a C₁-C₄ alkoxy radical, preferably methoxy; or OH; A represents a linear or branched, C₃-C₈ and preferably C₃-C₆ alkylene radical; m and n are integers dependent on the molecular weight and whose sum is between 1 and 2000.

According to a first embodiment, R, R' and R", which may be identical or different, represent a C₁-C₄ alkyl or hydroxyl radical, A represents a C₃ alkylene radical and m and n are such that the weight-average molecular mass of the compound is between 5000 and 500 000 approximately. Compounds of this type are referred to in the CTFA dictionary as "amodimethicones".

According to a second embodiment, R, R' and R", which may be identical or different, represent a C₁-C₄ alkoxy or hydroxyl radical, at least one of the radicals R or R" is an alkoxy radical and A represents a C₃ alkylene radical. The hydroxy/alkoxy mole ratio is preferably between 0.2/1 and 0.4/1 and advantageously equal to 0.3/1. Moreover, m and n are such that the weight-average molecular mass of the compound is between 2000 and 10⁶. More particularly, n is between 0 and 999 and m is between 1 and 1000, the sum of n and m being between 1 and 1000.

In this category of compounds, mention may be made, inter alia, of the product Belsil® ADM 652 sold by Wacker.

According to a third embodiment, R and R", which are different, represent a C₁-C₄ alkoxy or hydroxyl radical, at least one of the radicals R or R" is an alkoxy radical, R' represents a methyl radical and A represents a C₃ alkylene radical. The hydroxy/alkoxy mole ratio is preferably between 1/0.8 and 1/1.1 and advantageously equal to 1/0.95. Moreover, m and n are such that the weight-average molecular mass of the compound is between 2000 and 200 000. More particularly, n is between 0 and 999 and m is between 1 and 1000, the sum of n and m being between 1 and 1000.

More particularly, mention may be made of the product Fluid WR® 1300 sold by Wacker.

According to a fourth embodiment, R and R" represent a hydroxyl radical, R' represents a methyl radical and A is a C₄-C₈ and preferably C₄ alkylene radical. Moreover, m and n are such that the weight-average molecular mass of the compound is between 2000 and 10⁶. More particularly, n is between 0 and 1999 and m is between 1 and 2000, the sum of n and m being between 1 and 2000.

A product of this type is especially sold under the name DC 28299 by Dow Corning.

Note that the molecular mass of these silicones is determined by gel permeation chromatography (ambient temperature, polystyrene standard; µ styragem columns; eluent THF; flow rate 1 mm/m; 200 µl of a solution containing 0.5% by weight of silicone in THF are injected, and detection is performed by refractometry and UV-metry).

A product corresponding to the definition of formula (IA) is in particular the polymer known in the CTFA dictionary (7th edition, 1997) as "trimethylsilyl amodimethicone", corresponding to formula (IB) below: 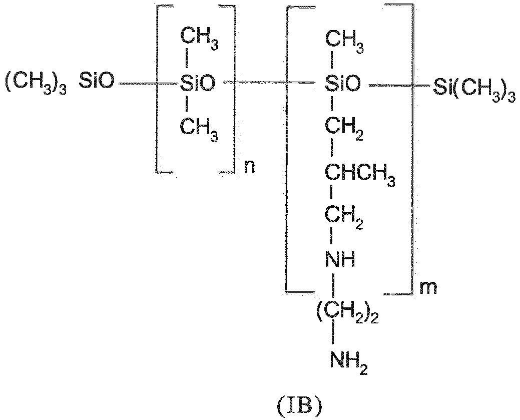 in which n and m have the meanings given above in accordance with formula (I) or (IA).

Such compounds are described, for example, in EP 0 095 238; a compound of formula (IB) is sold, for example, under the name Q2-8220 by the company OSI.
(b) the compounds corresponding to formula (II) below: 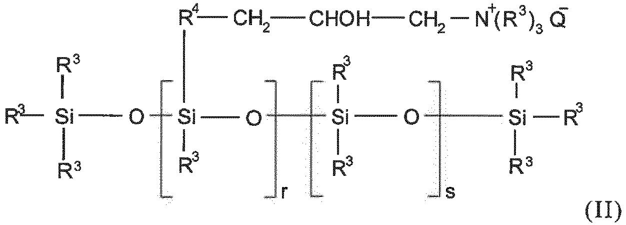 in which:
R³ represents a C₁-C₁₈ monovalent hydrocarbon-based radical, and in particular a C₁-C₁₈ alkyl or C₂-C₁₈ alkenyl radical, for example methyl;
R⁴ represents a divalent hydrocarbon-based radical, especially a C₁-C₁₈ alkylene radical or a divalent C₁-C₁₈, and for example C₁-C₈, alkylenoxy radical;
Q⁻ is a halide ion, in particular chloride;
r represents a mean statistical value from 2 to 20 and in particular from 2 to 8;
s represents a mean statistical value from 20 to 200 and in particular from 20 to 50.

Such compounds are described more particularly in patent US-4 185 087.

A compound falling within this class is the product sold by the company Union Carbide under the name Ucar Silicone ALE 56.
(c) the quaternary ammonium silicones of formula (III): 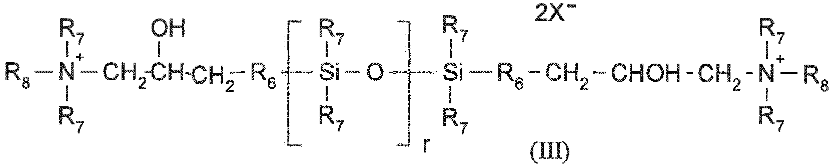 in which:
R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example methyl;
R6 represents a divalent hydrocarbon-based radical, especially a C₁-C₁₈ alkylene radical or a divalent C₁-C₁₈, and for example C₁-C₈, alkylenoxy radical linked to the Si via an SiC bond;
R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
X⁻ is an anion such as a halide ion, especially chloride, or an organic acid salt (acetate, etc.); ;
r represents a mean statistical value from 2 to 200 and in particular from 5 to 100;

These silicones are described, for example, in patent application EP-A 0 530 974.
(d) the amino silicones of the following formula (IV): 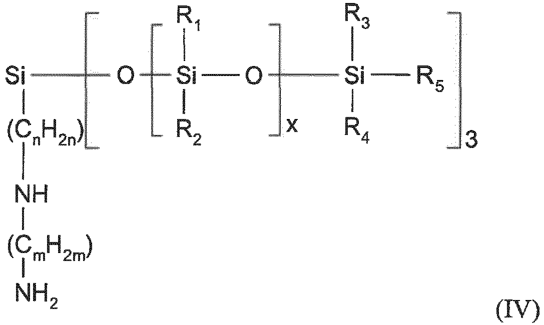 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5, and
in which x is chosen such that the amine number is between 0.01 and 1 meq/g.

(e) multiblock polyoxyalkylenated amino silicones, of the type (AB)n, A being a polysiloxane block and B being a polyoxyalkylene block comprising at least one amine group.

The said silicones preferably consist of repeating units having the following general formulae:

[-(SiMe₂O)ₓSiMe₂ - R -N(R")- R'-O(C₂H₄O)ₐ(C₃H₆O)_{b} -R'-N(H)-R-]

or alternatively

[-(SiMe₂O)ₓSiMe₂ - R -N(R")- R'-O(C₂H₄O)ₐ(C₃H₆O)_{b}-]

in which:
- a is an integer greater than or equal to 1, preferably ranging from 5 to 200 and more particularly ranging from 10 to 100;
- b is an integer between 0 and 200, preferably ranging from 4 to 100 and more particularly ranging from 5 to 30;
- x is an integer ranging from 1 to 10 000 and more particularly from 10 to 5000;
- R" is a hydrogen atom or a methyl;
- R, which may be identical or different, represent a linear or branched C₂-C₁₂ divalent hydrocarbon-based radical, optionally comprising one or more heteroatoms such as oxygen; preferably, R denotes an ethylene radical, a linear or branched propylene radical, a linear or branched butylene radical, or a -CH₂CH₂CH₂OCH(OH)CH₂-radical; preferentially, R denotes a -CH₂CH₂CH₂OCH(OH)CH₂-radical;
- R', which may be identical or different, represent a linear or branched C₂-C₁₂ divalent hydrocarbon-based radical, optionally comprising one or more heteroatoms such as oxygen; preferably, R' denotes an ethylene radical, a linear or branched propylene radical, a linear or branched butylene radical, or a -CH₂CH₂CH₂OCH(OH)CH₂-radical; preferentially, R' denotes -CH(CH₃)-CH₂-.

The siloxane blocks preferably represent from 50 mol% to 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular weight (Mw) of the silicone is preferably between 5000 and 1 000 000 and more particularly between 10 000 and 200 000.

Mention may be made especially of the silicones sold under the names Silsoft A-843 or Silsoft A+ by Momentive.

The silicone(s) that are particularly preferred are the multiblock polyoxyalkylenated aminated silicones.

The amino silicone(s) used in the composition according to the invention may be present in a content ranging from 0,1% to 15% by weight, preferably in a content ranging from 0,5% to 12% by weight and better still in a content ranging from 1 % to 8% by weight relative to the total weight of the composition.

### Cationic surfactant

Cationic surfactants used in the composition of the present invention may be selected from salts of optionally polyoxyalkylenated primary, secondary or tertiary fatty amines, quaternary ammonium salts, and mixtures thereof.

The cationic surfactants used in the composition of the present invention are preferably quaternary ammonium salts.

Quaternary ammonium salts include especially, for example:
- those corresponding to the general formula (VI) below: 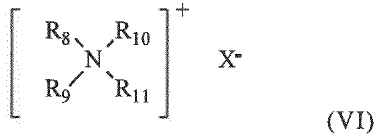 in which the radicals R₈ to R₁₁, which may be identical or different, represent a linear or branched aliphatic radical comprising from 1 to 30 carbon atoms, or an aromatic radical such as aryl or alkylaryl, with at least one of the radicals R₈ to R₁₁ denoting a linear or branched alkyl radical comprising from 10 to 30 carbon atoms, and X- denoting an organic or inorganic anion. The aliphatic radicals may include heteroatoms such as, in particular, oxygen, nitrogen, sulfur and halogens. The aliphatic radicals are selected for example from C₁₋₃₀ alkyl, C₁₋₃₀ alkoxy, polyoxyalkylene (C₂-C₆), C₁₋₃₀ alkylamide, alkyl(C₁₂-C₂₂)amidoalkyl(C₂-C₆) and C₁₋₃₀ hydroxyalkyl radicals; X is an anion selected from the group of halides, phosphates, acetates, lactates, alkyl(C₂-C₆)sulfates and alkyl- or alkylaryl-sulfonates.

Among the quaternary ammonium salts of formula (VI), preference is firstly given to tetraalkylammonium chlorides such as, for example, dialkyldimethylammonium or alkyltrimethylammonium chlorides in which the alkyl radical comprises approximately from 12 to 22 carbon atoms, more particularly behenyltrimethylammonium, distearyldimethylammonium, cetyltrimethylammonium, and benzyldimethylstearylammonium chlorides, or else, secondly, to palmitylamidopropyltrimethylammonium chloride or stearamidopropyldimethyl(myristyl acetate)-ammonium chloride, which is sold under the name Ceraphyl® 70 by the company Van Dyk.
- quaternary ammonium salts of imidazoline, such as, for example, those of formula (VII) below: 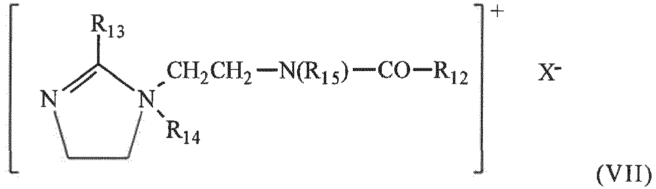 in which R₁₂ represents an alkenyl or alkyl radical comprising from 8 to 30 carbon atoms, derived for example from tallow fatty acids, R₁₃ represents a hydrogen atom, a C₁-C₄ alkyl radical or an alkenyl or alkyl radical comprising from 8 to 30 carbon atoms, R₁₄ represents a C₁-C₄ alkyl radical, R₁₅ represents a hydrogen atom or a C₁-C₄ alkyl radical, X⁻ is an anion selected from the group of halides, phosphates, acetates, lactates, alkyl sulfates and alkyl- or alkylaryl-sulfonates. Preferably, R₁₂ and R₁₃ denote a mixture of alkenyl or alkyl radicals comprising from 12 to 21 carbon atoms, for example derived from tallow fatty acids, R₁₄ denotes a methyl radical and R₁₅ denotes a hydrogen atom. A product of this kind is sold for example under the name Rewoquat® W 75 by the company Rewo;
- quaternary diammonium or triammonium salts, particularly of formula (VIII) below: 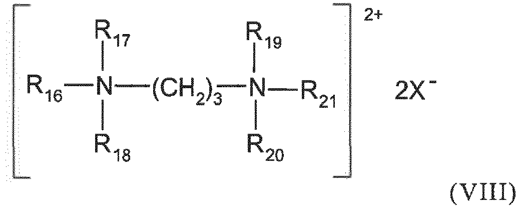 in which formula (VIII):
   R₁₆ denotes an alkyl group comprising approximately from 16 to 30 carbon atoms, which is optionally hydroxylated and/or interrupted with one or more oxygen atoms;
   R₁₇ is selected from hydrogen, an alkyl group comprising from 1 to 4 carbon atoms or a group -(CH₂)₃-N⁺(R₁₆ₐ)(R₁₇ₐ)(R₁₈ₐ), X⁻;
   R₁₆ₐ, R₁₇ₐ, R₁₈ₐ, R₁₈, R₁₉, R₂₀ and R₂₁, which may be identical or different, are selected from hydrogen and an alkyl group comprising from 1 to 4 carbon atoms; and
   X⁻, which may be identical or different, represent an organic or inorganic anionic counterion, such as that selected from halides, acetates, phosphates, nitrates, alkyl(C₁-C₄) sulfates, alkyl(C₁-C₄)- or alkyl(C₁-C₄)aryl-sulfonates, more particularly methyl sulfate and ethyl sulfate.

Compounds of this kind are, for example, Finquat CT-P, available from the company Finetex (Quaternium 89), and Finquat CT, available from the company Finetex (Quaternium 75);
- -quaternary ammonium salts containing one or more ester functions, such as those of formula (IX) below: 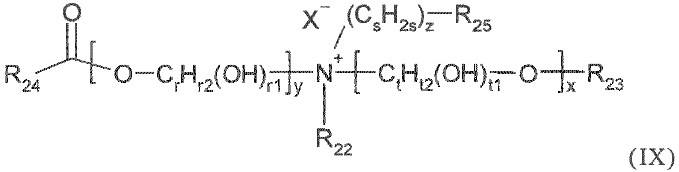 in which formula (IX):
   R₂₂ is selected from C₁-C₆ alkyl groups and C₁-C₆ hydroxyalkyl or C₁-C₆ dihydroxyalkyl groups,
   R₂₃ is selected from:
- the group 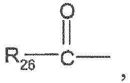
- linear or branched, saturated or unsaturated C₁-C₂₂ hydrocarbon groups R₂₇,
- hydrogen atom,
   R₂₅ is selected from:
- the group 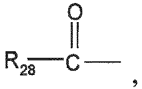
- linear or branched, saturated or unsaturated C₁-C₆ hydrocarbon groups R₂₉,
- hydrogen atom,
   R₂₄, R₂₆ and R₂₈, which are identical or different, are selected from linear or branched, saturated or unsaturated C₇-C₂₁ hydrocarbon groups;
   r, s and t, which are identical or different, are integers from 2 to 6,
   r1 and t1, which are identical or different, are equal to 0 or 1, with r2+r1=2r and t1+t2=2t,
   y is an integer ranging from 1 to 10,
   x and z, which are identical or different, are integers from 0 to 10,
   X⁻ represents an organic or inorganic anionic counterion,
   with the proviso that the sum x + y + z equals from 1 to 15, that, when x is 0, then R₂₃ denotes R₂₇ and that, when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z has a value from 1 to 10.

When R₂₃ is an R₂₇ hydrocarbon group, it may be long and may have from 12 to 22 carbon atoms, or may be short and may have from 1 to 3 carbon atoms.

When R₂₅ is an R₂₉ hydrocarbon group, it preferably has 1 to 3 carbon atoms.

Advantageously, R₂₄, R₂₆ and R₂₈, which are identical or different, are selected from linear or branched, saturated or unsaturated C₁₁-C₂₁ hydrocarbon groups, and more particularly from linear or branched, saturated or unsaturated C₁₁-C₂₁ alkyl and alkenyl groups.

Preferably, x and z, which are identical or different, are equal to 0 or 1.

Advantageously, y is 1.

Preferably, r, s and t, which are identical or different, are 2 or 3, and more particularly they are 2.

The anionic counterion X⁻ is preferably a halide, such as chloride, bromide or iodide; a alkyl(C₁-C₄) sulfate or a alkyl(C₁-C₄)- or alkyl(C₁-C₄)aryl-sulfonate. It is possible, however, to use methanesulfonate, phosphate, nitrate or tosylate, an anion derived from organic acid such as acetate or lactate, or any other anion compatible with the ester-functional ammonium.

The anionic counterion X⁻ is even more particularly chloride, methyl sulfate or ethyl sulfate.

Use is made more particularly, in the composition according to the invention, of the ammonium salts of formula (VI) in which:
- R₂₂ denotes a methyl or ethyl group,
- x and y are equal to 1,
- z is equal to 0 or 1,
- r, s and t are equal to 2,
- R₂₃ is selected from:
   the group 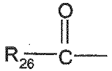 methyl, ethyl or C₁₄-C₂₂ hydrocarbon groups,
   a hydrogen atom,
- R₂₅ is selected from:
   the group 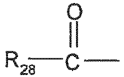 a hydrogen atom,
- R₂₄, R₂₆ and R₂₈, which may be identical or different, are selected from linear or branched, saturated or unsaturated C₁₃-C₁₇ hydrocarbon groups, and preferably from linear or branched, saturated or unsaturated C₁₃-C₁₇ alkyl and alkenyl groups.

Advantageously, the hydrocarbon radicals are linear.

Mention may be made, for example, among the compounds of formula (IX), of diacyloxyethyldimethylammonium, diacyloxy-ethylhydroxyethylmethylammonium, monoacyloxyethyldihydroxyethylmethylammonium, triacyloxyethyl-methylammonium and monoacyloxyethylhydroxyethyl-dimethylammonium salts, especially the chloride or the methyl sulfate, and mixtures of these compounds. The acyl groups preferably have 14 to 18 carbon atoms and originate more particularly from a vegetable oil such as palm oil or sunflower oil. When the compound contains a plurality of acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca, Rewoquat® WE 18 by the company Rewo-Witco.

The composition according to the invention may contain, for example, a mixture of quaternary ammonium monoester, diester and triester salts, with a majority by weight of diester salts.

It is also possible to use the ammonium salts containing at least one ester function that are described in patents US-A-4874554 and US-A-4137180.

Use may be made of the behenoyl-hydroxypropyltrimethylammonium chloride available from Kao under the name Quatarmin BTC 131.

Preferably, the ammonium salts containing at least one ester function contain two ester functions.

Among the quaternary ammonium salts containing at least one ester function that can be used, it is preferred to use dipalmitoylethylhydroxyethylmethylammonium salts.

The cationic surfactants used in the composition are preferably selected from quaternary ammonium salts of formula (III) and quaternary ammonium salts comprising at least one ester function, especially those selected from those corresponding to the formula (VI).

The cationic surfactants used in the composition are preferably selected from behenyltrimethylammonium, distearyldimethylammonium, cetyltrimethylammonium and benzyldimethylstearylammonium chlorides, or else from palmitylamidopropyltrimethylammonium or stearamidopropyldimethyl(myristyl acetate)-ammonium chlorides.

More preferably still, the cationic surfactant is cetyltrimethylammonium chloride or behenyltrimethylammonium chloride

Cationic surfactants of the invention are different from ingredients (i). Preferably, they are non siliconated.

The term non siliconated means that there is no siloxane group Si-O in the chemical structure of the surfactant.

The cationic surfactants used in the composition of the present invention may be present in said composition in an amount of from 0,1 wt% to 20% by wt%, more preferably from 0,5 to 15% by wt%, preferably from 1 by wt% to 10 by wt%, relative to the total weight of the composition.

### Associative polymer

Associative polymers are water-soluble or water dispersible polymers that are capable, in an aqueous medium, of reversibly combining with each other or with other molecules.

The associative polymer used in the invention is an amphiphilic polymer, which means a polymer comprising at least one hydrophilic moiety which renders the polymer soluble in water and at least one hydrophobic region, comprising at least one fatty chain, by means of which the polymer interacts and undergoes assembly with another associative polymer or with other molecules.

Hence the associative polymer used in the present invention is preferably an amphiphilic polymer which comprises at least one hydrophilic group and at least one fatty chain.

According to the present invention, a fatty chain has at least 8 carbon atoms, preferably from 8 to 30 carbon atoms, more preferably from 10 to 30 carbon atoms and especially from 12 to 22 carbon atoms.

According to the present invention the associative polymer has at least one repetitive unit other than an oxyalkylenated group. So associative polymers are different from products resulting merely from the condensation of an alkylene oxide with an alcohol, an ester or an amide.

The associative polymers of the present invention are cationic associative polymers chosen from quaternized hydroxyethylcelluloses modified with at least one alkyl group containing at least 8 carbon atoms.

### Cationic associative polymer

The associative polymer is chosen from cationic amphiphilic polymer.

The associative cationic amphiphilic polymers are chosen from:
- (2) quaternized cellulose derivatives,

The quaternized cellulose derivatives are quaternized hydroxyethylcelluloses modified with groups comprising at least one alkyl group comprising at least 8 carbon atoms.

The alkyl radicals borne by the above hydroxyethylcelluloses preferably contain from 8 to 30 carbon atoms, especially from 10 to 30 carbon atoms.

Examples of quaternized alkylhydroxyethylcelluloses containing C₈-C₃₀ fatty chains that may be mentioned include the products PQ-67 ® (C₁₂ alkyl), Quatrisoft LM 200®, Quatrisoft LM-X 529-18-A®, Quatrisoft LM-X 529-18B® (C₁₂ alkyl) and Quatrisoft LM-X 529-8® (C₁₈ alkyl) or Softcat Polymer SL100 (C₁₂ alkyl) sold by the company Amerchol, and the products Crodacel QM®, Crodacel QL® (C₁₂ alkyl) and Crodacel QS® (C₁₈ alkyl) sold by the company Croda.

In particular, the cationic associative polymer is a cationic amphiphilic polymer having at least one fatty chain comprising at least 8 carbon atoms, especially from 10 to 30 carbon atoms and more specifically from 10 to 22 carbon atoms.

The associative polymer is chosen from quaternized hydroxyethylcelluloses modified with at least an alkyl group containing at least 8 carbon atoms, especially from 10 to 22 carbon atoms and more specifically from 10 to 16 carbon atoms.

The associative polymer is especially an associative cationic polymer having the INCI name POLYQUATERNIUM-67.

The associative(s) polymer(s) used in the present invention is/are present in said composition in an amount of from 0.01 wt% to 5 wt%, preferably from 0.05 to 2 wt%, more preferably from 0.1 wt% to 1 wt%, relative to the total weight of the composition.

### Thickening agent

The composition according to the invention further comprises one or more thickeners agents different from the associative polymers previously described.

For the purposes of the present invention, the term "thickener" means any compound whose presence increases the viscosity of the composition into which it is introduced by at least 25 cps and preferably 50 cps at 25°C and at a shear rate of 1 s⁻¹.

Preferably, the thickener agent according to the invention is non ionic.

The thickener(s) may preferably be chosen from fatty alcohols, fatty acid amides, oxyalkylenated fatty acid esters and thickening polymers, or mixtures thereof.

### Fatty alcohol

For the purposes of the present invention, the term "fatty alcohol" means any saturated or unsaturated, linear or branched alcohol containing at least 8 carbon atoms.

The fatty alcohol may have the structure R-OH in which R denotes a saturated or unsaturated, linear or branched radical containing from 8 to 40 and preferably from 8 to 30 carbon atoms; R preferably denotes a C₈-C₄₀ and preferably C₁₂-C₂₄ alkyl or a C₈-C₄₀ and preferably C₁₂-C₂₄ alkenyl group. R may be substituted with one or more hydroxyl groups and especially with one or two hydroxyl groups.

Examples that may be mentioned include lauryl alcohol, cetyl alcohol, stearyl alcohol, oleyl alcohol, behenyl alcohol, linoleyl alcohol, undecylenyl alcohol, palmitoleyl alcohol, arachidonyl alcohol and erucyl alcohol, and mixtures thereof.

The fatty alcohol may represent a mixture of fatty alcohols, which means that several species of fatty alcohol may coexist, in the form of a mixture, in a commercial product.

Advantageously, the fatty alcohol is solid or pasty at a temperature of 25°C. For the purposes of the present invention, the term "fatty alcohol that is solid or pasty at 25°C" means a fatty alcohol that has a viscosity, measured with a rheometer (for example an R600 rheometer) at a shear rate of 1 s⁻¹, of greater than or equal to 1 Pa.s.

Preferably, the fatty alcohols used in the composition according to the invention are stearyl alcohol, cetyl alcohol and mixtures thereof, such as cetylstearyl alcohol.

Even more preferentially, the fatty alcohol used in the composition according to the invention is cetyl alcohol.

### Fatty acid amides

For the purposes of the present invention, the term "fatty acid amide" means an amide comprising in its structure at least one hydrocarbon-based chain comprising at least 8 carbon atoms.

The fatty acid amides are more particularly chosen from compounds derived from an amide of an alkanolamine and of a saturated or unsaturated, linear or branched C₈-C₃₀ fatty acid, the alkanolamine and/or the fatty acid being optionally oxyalkylenated and more particularly oxyethylenated with 1 to 50 mol of ethylene oxide.

Preferably, the fatty acid amides are chosen from amides of a C₂-C₁₀ alkanolamine and of a C₁₄-C₃₀ fatty acid, and even more preferentially from amides of a C₂-C₁₀ alkanolamine and of a C₁₄-C₂₂ fatty acid.

Advantageously, the fatty acid amide is chosen from:
- coconut acid monoisopropanolamide, such as the amide sold under the trade name Empilan CLS by the company Huntsman,
- oleic acid diethanolamide, such as the amide sold under the trade name Mexanyl® GT by the company Chimex,
- myristic acid monoethanolamide, such as the amide sold under the trade name Comperlan® MM by the company Cognis,
- soybean fatty acid diethanolamide, such as the amide sold under the trade name Comperlan® VOD by the company Cognis,
- stearic acid ethanolamide, such as the amide sold under the trade name Monamid® S by the company Uniqema,
- oleic acid monoisopropanolamide, such as the amide sold under the trade name Witcamide® 61 by the company Witco,
- linoleic acid diethanolamide, such as the amide sold under the trade name Purton® SFD by the company Zschimmer Schwarz,
- stearic acid monoethanolamide, such as the amide sold under the trade name Monamid® 972 by the company ICI/Uniqema,
- behenic acid monoethanolamide, such as the amide sold under the trade name Incromide® BEM from Croda,
- isostearic acid monoisopropanolamide, such as the amide sold under the trade name Witcamide® SPA by the company Witco,
- erucic acid diethanolamide, such as the amide sold under the trade name erucic acid diethanolamide by the company Stéarineries Dubois,
- ricinoleic acid monoethanolamide, such as the amide sold under the trade name ricinoleic monoethanolamide by the company Stéarineries Dubois,
- rapeseed fatty acid amide containing 4 mol of ethylene oxide, such as the product sold under the name Amidet N by the company Kao.

### Oxyalkylenated fatty acid esters

The nonionic thickener(s) may be chosen from oxyalkylenated derivatives of fatty acid esters or of fatty alcohol ethers.

Oxyalkylenated derivatives of fatty acid esters that may especially be mentioned include ethoxylated alkyl or acyl derivatives of polyols, which may in particular be oxyethylenated derivatives of C₆-C₃₀ fatty acid esters or of C₆-C₃₀ fatty alcohol ethers, and of polyols such as glycerol, sorbitol, glucose, pentaerythritol or polyethylene glycol, more particularly polyethylene glycol, these oxyethylenated derivatives generally comprising from 50 to 500 oxyethylene groups and preferably from 100 to 300 oxyethylene groups.

Examples of compounds of this type that may be mentioned include ethylene glycol stearate, polyethylene glycol distearate comprising 150 oxyethylene groups (150 OE), oxyethylenated (200 OE) glyceryl stearate, such as the product sold under the name Simulsol 220 TM® by the company SEPPIC, oxyethylenated (150 OE) pentaerythrityl tetrastearate, such as the product sold under the name Crothix® by the company Croda, oxyethylenated (120 OE) methylglucose dioleate, such as the product sold under the name Glucamate DOE-120 Vegetal® by the company Amerchol, oxyethylenated (160 OE) sorbitan triisostearate such as the product sold under the name Rheodol TW IS399C by the company Kao Chemicals, and oxyethylenated (55 ethylene oxide) propylene glycol oleate, such as the product sold under the reference Antil 141 Liquid by the company Evonik Goldschmidt, and mixtures thereof.

In one variant of the invention, the oxyethylenated fatty acid esters are of formula (A) below:

R₁-CO(X)ₙ-(OCH₂CH₂)ₘ-O-(CO)ₚ-R₂ (A)

X denoting a linear or branched C₁-C₄ alkylene radical, and preferably the radical having the following formula: 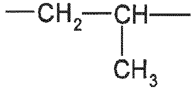
n denoting 0 or 1,
p denoting 0 or 1,
m ranging from 50 to 200, and
R₁ denoting a linear or branched C₉-C₂₉ alkyl or alkenyl radical and R² denoting a hydrogen atom or a linear or branched C₉-C₂₉ alkyl or alkenyl radical.

More particularly, among these esters, polyethylene glycol distearate comprising 150 oxyethylene groups (150 OE) is preferred.

### Thickening polymers

The thickening polymers of the invention are different from the amides and esters already described and also from products resulting merely from the condensation of an alkylene oxide with an alcohol, an ester or an amide.

In particular, the nonionic thickening polymers are non-associative.

According to the invention, the term "non-associative thickening polymer" means a thickening polymer not simultaneously comprising at least one C₈-C₃₀ fatty chain and at least one hydrophilic unit.

Preferably the thickening polymers of the invention are non ionic.

The nonionic non-associative thickening polymers according to the invention may be of natural or synthetic origin. They are chosen especially from:
(i) nonionic homopolymers and copolymers containing ethylenically unsaturated monomers of ester and/or amide type,
(ii) vinylpyrrolidone homopolymers or copolymers,
(iii) polysaccharides.

Among the nonionic homopolymers or copolymers containing ethylenically unsaturated monomers of ester and/or amide type that may be mentioned are polyamides, especially the products sold under the names: Cyanamer P250 by the company Cytec (polyacrylamide); methyl methacrylate/ethylene glycol dimethacrylate copolymers (PMMA MBX-8C by the company US Cosmetics); butyl methacrylate/methyl methacrylate copolymers (Acryloid B66 by the company Röhm & Haas); polymethyl methacrylate (BPA 500 by the company Kobo).

The vinylpyrrolidone homopolymers or copolymers are chosen especially from crosslinked vinylpyrrolidone homopolymers such as the Polymer ACP-10 sold by ISP.

The thickening polysaccharides are especially chosen from glucans, modified or unmodified starches (such as those derived, for example, from cereals, for instance wheat, corn or rice, from vegetables, for instance yellow pea, and tubers, for instance potato or cassava), amylose, amylopectin, glycogen, dextrans, celluloses and derivatives thereof (methylcelluloses, hydroxyalkylcelluloses, ethylhydroxyethylcelluloses), mannans, xylans, lignins, arabans, galactans, galacturonans, chitin, chitosans, glucuronoxylans, arabinoxylans, xyloglucans, glucomannans, pectic acids and pectins, arabinogalactans, carrageenans, agars, gum arabics, gum tragacanths, ghatti gums, karaya gums, carob gums, galactomannans such as guar gums and nonionic derivatives thereof (hydroxypropyl guar), and mixtures thereof.

In general, the compounds of this type that may be used in the present invention are chosen from those described especially in Kirk-Othmer's Encyclopedia of Chemical Technology, Third Edition, 1982, volume 3, pp. 896-900, and volume 15, pp. 439-458, in Polymers in Nature by E.A. MacGregor and C.T. Greenwood, published by John Wiley & Sons, Chapter 6, pp. 240-328, 1980, and in Industrial Gums - Polysaccharides and their Derivatives, edited by Roy L. Whistler, Second Edition, published by Academic Press Inc., the content of these three publications being entirely included in the present patent application by way of reference.

Starches, guar gums and celluloses and derivatives thereof will preferably be used.

The polysaccharides as mentioned before can be modified or unmodified.

The unmodified guar gums are, for example, the products sold under the name Vidogum GH 175 by the company Unipectine and under the names Meypro-Guar 50 and Jaguar C by the company Rhodia Chimie.

The modified nonionic guar gums are especially modified with C₁-C₆ hydroxyalkyl groups.

Among the hydroxyalkyl groups that may be mentioned, for example, are hydroxymethyl, hydroxyethyl, hydroxypropyl and hydroxybutyl groups.

These guar gums are well known in the prior art and can be prepared, for example, by reacting the corresponding alkene oxides such as, for example, propylene oxides, with the guar gum so as to obtain a guar gum modified with hydroxypropyl groups.

The degree of hydroxyalkylation, which corresponds to the number of alkylene oxide molecules consumed by the number of free hydroxyl functions present on the guar gum, preferably ranges from 0.4 to 1.2.

Such nonionic guar gums optionally modified with hydroxyalkyl groups are sold, for example, under the trade names Jaguar HP8, Jaguar HP60 and Jaguar HP120, Jaguar DC 293 and Jaguar HP 105 by the company Rhodia Chimie or under the name Galactasol 4H4FD2 by the company Aqualon.

Among the celluloses that are especially used are hydroxyethylcelluloses and hydroxypropylcelluloses. Mention may be made of the products sold under the names Klucel EF, Klucel H, Klucel LHF, Klucel MF and Klucel G by the company Aqualon, and Cellosize Polymer PCG-10 by the company Amerchol.

In a preferred embodiment, the thickening agents used in the composition of the present invention are nonionic.

In a preferred embodiment, the thickening agents used in the composition are non ionic agents chosen from fatty alcohols, non ionic non-associative polymers and mixture thereof.

Particularly, the thickening agents are chosen from fatty alcohols and polysaccharides and more particularly from fatty alcohols and non ionic polysaccharides.

The thickening agents used in the present invention may be present in said composition in an amount of from 0,1 wt% to 15 by wt%, more preferably from 0.5 to 10 by wt%, preferably from 1 by wt% to 8 by wt%, relative to the total weight of the composition.

The compositions of the invention preferably comprise water or a mixture of water and one or more organic solvents chosen from C₁-C₄ lower alcohols, such as ethanol, isopropanol, tert-butanol or n-butanol; polyols such as glycerol, propylene glycol and polyethylene glycols; and mixtures thereof.

The composition according to the invention comprises an amount of water which is generally greater than or equal to 5% by weight, relative to the total weight of the composition, preferably an amount of water greater than or equal to 20% by weight relative to the total weight of the composition.

Preferably, the amount of water in the composition according to the invention is less than or equal to 95% and preferentially less than or equal to 90% by weight relative to the total weight of the composition. The organic solvents may be present in a concentration ranging from 0.1% to 40% and better still from 1% to 20% by weight relative to the total weight of the composition.

The pH of the compositions according to the invention generally ranges from 3 to 9, most preferably from 4 to 7.

The composition according to the invention may also comprise one or more standard additives that are well known in the art, chosen from surfactants other than cationic surfactants previously described, particularly non ionic surfactants, moisturizers; emollients, plasticizers, permanent or temporary dyes, fragrances, peptizers, preserving agents, active agents, ceramides or pseudoceramides; oils and waxes, vitamins or provitamins; pH stabilizers, preserving agents; proteins, sequestrants; solubilizers; reducing agents or antioxidants; oxidizing agents; basifying agents, acidifying agents, anticorrosion agents and non-thickening cationic polymers, and mixtures thereof.

A person skilled in the art will take care to select the optional standard additives and the amount thereof such that they do not harm the properties of the compositions of the present invention.

These standard additives are generally present in the composition according to the invention in an amount ranging from 0 to 20% by weight relative to the total weight of the composition.

The composition according to the invention may be in the form of an emulsion. More specifically, it may be either in the form of an oil-in-water emulsion, with the continuous phase being the aqueous phase, or in the form of a water-in-oil emulsion, with the continuous phase being the fatty phase.

The compositions in accordance with the invention may be used for conditioning keratin fibres, in particular the hair, for example as hair conditioners.

Preferably, the compositions of the invention are rinse-off or leave-in hair conditioners.

Another subject of the present invention is a process for treating keratin fibres, such as the hair, which consists in applying a composition as described above on the said fibres.

The compositions described above may be used on any type of hair: light or dark hair, natural hair or hair that has undergone a cosmetic treatment such as permanent waving, dyeing, bleaching or relaxing.

In a preferred embodiment, the composition of the present invention is applied on sensitized hair.

The application to the hair of the composition according to the invention may be performed, for example, using a comb, a fine brush, a coarse brush or with the fingers.

According to one particular embodiment of the invention, the application of the composition is followed by drying at room temperature or at a temperature above 40°C.

The drying may be performed immediately after the application or after a leave-on time that may range from 1 minute to 30 minutes.

Preferably, the hair is dried, in addition to using a supply of heat, with a flow of air. This flow of air during drying makes it possible to improve the individualization of the coating.

During drying, a mechanical action may be exerted on the locks, such as combing, brushing or by running the fingers through the hair.

The drying step of the process of the invention may be performed with a hood, a hair dryer, a straightening iron, etc.

When the drying step is performed with a hood or a hair dryer, the drying temperature is between 40 and 110°C and preferably between 50 and 90°C.

When the drying step is performed with a straightening iron, the drying temperature is between 110 and 230°C and preferably between 130 and 200°C.

The invention also relates to the use of the composition according to the invention as a hair conditioner, preferably used after the application of a shampoo or shampoo and other conditioner.

The examples below are given as illustrations of the present invention.

### EXAMPLE

Concentrations are given as active raw material.

| Nom INCI US | Concentration (active raw material) |
|---|---|
| POLYQUATERNIUM-67 | 0.237 |
| HYDROXYETHYLCELLULOSE | 0.5 |
| CHLORHEXIDINE DIGLUCONATE | 0.04 |
| CETEARYL ALCOHOL | 3.0 |
| CITRIC ACID | 0.08 |
| BEHENTRIMONIUM CHLORIDE | 3.16 |
| PEG-40/PPG-8 METHYLAMINOPROPYL/HYDROXYP ROPYL DIMETHICONE COPOLYMER | 1.2 |
| FRAGRANCE | 0.4 |
| WATER | Qsp 100 |

The composition is applied on hair washed by a shampoo. After final rinsing, hair is softer, smoother and suppler and the number of splits ends is also reduced.

## Claims

1. Composition comprising:
(i) one or more amino silicone,
(ii) one or more cationic surfactants different from (i),
(iii) one or more associative polymers, and
(iv) one or more thickening agents different from the associative polymers (iii),
the associative polymers being cationic amphiphilic associative polymers chosen from quaternized hydroxyethylcelluloses modified with at least one alkyl group containing at least 8 carbon atoms,
the associative polymers being present in said composition in an amount of from 0.01 wt% to 5 wt%, relative to the total weight of the composition.

2. Composition according to claim 1, **characterized in that** the amino silicones are chosen from:
(a) the compounds corresponding to formula (I) below:
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
in which:
T is a hydrogen atom or a phenyl, hydroxyl (-OH) or C₁-C₈ alkyl radical, and preferably methyl, or a C₁-C₈ alkoxy, preferably methoxy,
a denotes the number 0 or an integer from 1 to 3, and preferably 0,
b denotes 0 or 1, and in particular 1,
m and n are numbers such that the sum (n + m) ranges from 50 to 150, wherein n denotes a number from 49 to 149, and m denotes a number from 1 to 10;
R¹ is a monovalent radical of formula -C_{q}H_{2q}L in which q is a number from 2 to 8 and L is an optionally quaternized amino group chosen from the following groups:
- N(R²)-CH₂-CH₂-N(R²)₂;
- N(R²)₂; -N⁺(R²)₃Q⁻' such as -N⁺(R²)(H)₂Q⁻, -N⁺(R²)₂HQ⁻;
- N(R²)-CH₂-CH₂-N⁺(R²)(H)₂Q⁻,
in which R² denotes a hydrogen atom, a phenyl, a benzyl or a saturated monovalent hydrocarbon-based radical, for example a C₁-C₂₀ alkyl radical, and Q⁻ represents a halide ion, for instance fluoride, chloride, bromide or iodide,
(b) the compounds corresponding to formula (II) below: 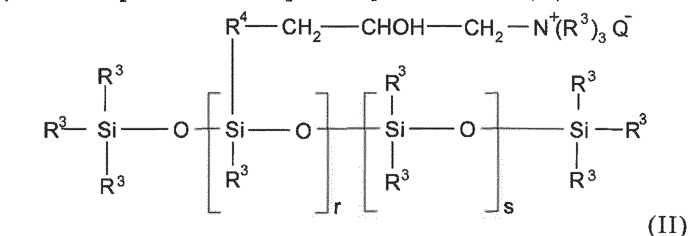 in which:
R³ represents a C₁-C₁₈ monovalent hydrocarbon-based radical, and in particular a C₁-C₁₈ alkyl or C₂-C₁₈ alkenyl radical, for example methyl;
R⁴ represents a divalent hydrocarbon-based radical, especially a C₁-C₁₈ alkylene radical or a divalent C₁-C₁₈, and for example C₁-C₈, alkylenoxy radical;
Q⁻ is a halide ion, in particular chloride;
r represents a mean statistical value from 2 to 20 and in particular from 2 to 8;
s represents a mean statistical value from 20 to 200 and in particular from 20 to 50,
(c) the quaternary ammonium silicones of formula (III): 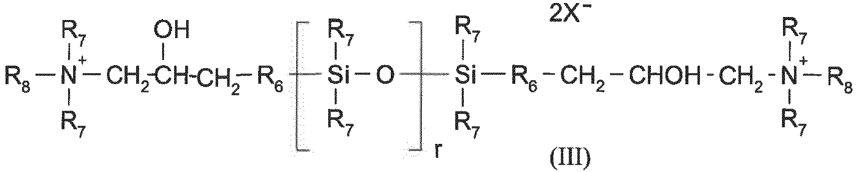 in which:
R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example methyl;
R₆ represents a divalent hydrocarbon-based radical, especially a C₁-C₁₈ alkylene radical or a divalent C₁-C₁₈, and for example C₁-C₈, alkylenoxy radical linked to the Si via an SiC bond;
R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
X⁻ is an anion such as a halide ion, especially chloride, or an organic acid salt (acetate, etc.);
r represents a mean statistical value from 2 to 200 and in particular from 5 to 100;
(d) the amino silicones of formula (IV) below: 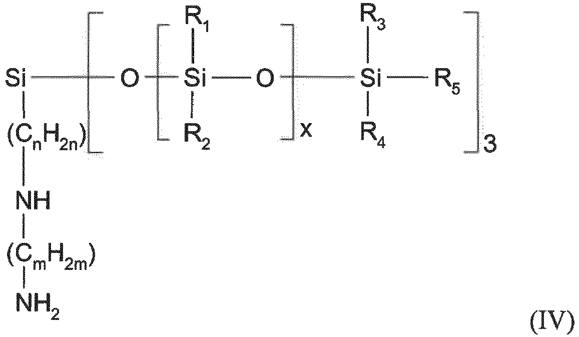 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5, and
in which x is chosen such that the amine number is between 0.01 and 1 meq/g.
(e) multiblock polyoxyalkylenated amino silicones, of the type (AB)n, A being a polysiloxane block and B being a polyoxyalkylene block comprising at least one amine group.

3. Composition according to claims 1 or 2, **characterized in that** the amino silicones are chosen from multiblock polyoxyalkylenated amino silicones, of the type (AB)n, A being a polysiloxane block and B being a polyoxyalkylene block comprising at least one amine group, silicones preferably consisting of repeating units having the following general formulae:
[-(SiMe₂O)ₓSiMe₂ - R -N(R")- R'-O(C₂H₄O)ₐ(C₃H₆O)_{b} -R'-N(H)-R-]
or alternatively
[-(SiMe₂O)ₓSiMe₂ - R -N(R")- R' - O(C₂H₄O)ₐ(C₃H₆O)_{b}-]
in which:
- a is an integer greater than or equal to 1, preferably ranging from 5 to 200 and more particularly ranging from 10 to 100;
- b is an integer between 0 and 200, preferably ranging from 4 to 100 and more particularly ranging from 5 to 30;
- x is an integer ranging from 1 to 10 000 and more particularly from 10 to 5000;
- R" is a hydrogen atom or a methyl;
- R, which may be identical or different, represent a linear or branched C₂-C₁₂ divalent hydrocarbon-based radical, optionally comprising one or more heteroatoms such as oxygen; preferably, R denotes an ethylene radical, a linear or branched propylene radical, a linear or branched butylene radical, or a -CH₂CH₂CH₂OCH(OH)CH₂-radical; preferentially, R denotes a -CH₂CH₂CH₂OCH(OH)CH₂-radical;
- R', which may be identical or different, represent a linear or branched C₂-C₁₂ divalent hydrocarbon-based radical, optionally comprising one or more heteroatoms such as oxygen; preferably, R' denotes an ethylene radical, a linear or branched propylene radical, a linear or branched butylene radical, or a -CH₂CH₂CH₂OCH(OH)CH₂-radical; preferentially, R' denotes -CH(CH₃)-CH₂-.

4. Composition according to anyone of the preceding claims, **characterized in that** the cationic surfactant(s) are chosen from salts of optionally polyoxyalkylenated primary, secondary or tertiary fatty amines, and quaternary ammonium salts, and mixtures thereof.

5. Composition according to anyone of the preceding claims, **characterized in that** the cationic surfactant is a quaternary ammonium salt chosen from:
- those that have the general formula (VI) below: 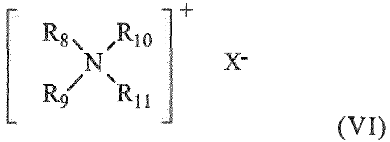 in which the radicals R₈ to R₁₁, which may be identical or different, represent a linear or branched aliphatic radical comprising from 1 to 30 carbon atoms, or an aromatic radical such as aryl or alkylaryl; X is an anion chosen from the group of halides, phosphates, acetates, lactates, (C₂-C₆)alkyl sulfates, and alkyl or alkylaryl sulfonates;
- quaternary ammonium salts of imidazoline;
- di- or triquaternary ammonium salts, in particular of formula (VIII): 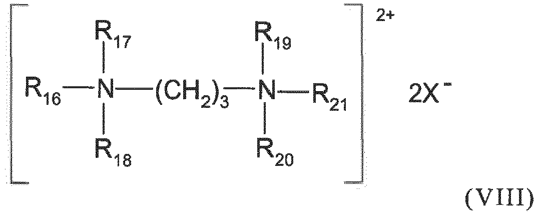 in which R₁₆ denotes an alkyl radical containing approximately from 16 to 30 carbon atoms, which is optionally hydroxylated and/or interrupted with one or more oxygen atoms and optionally branched, R₁₇ is selected from an alkyl radical containing from 1 to 4 carbon atoms or a group (R₁₆ₐ)(R₁₇ₐ)(R₁₈ₐ)N₊-(CH₂)₃-, R₁₆ₐ, R₁₇ₐ, R₁₈ₐ, R₁₈, R₁₉, R₂₀ and R₂₁, which may be identical or different, are selected from hydrogen and an alkyl radical containing from 1 to 4 carbon atoms, and X⁻ is an anion selected from the group of halides, acetates, phosphates, nitrates and methyl sulfates;
- quaternary ammonium salts containing at least one ester function of formula (IX) below: 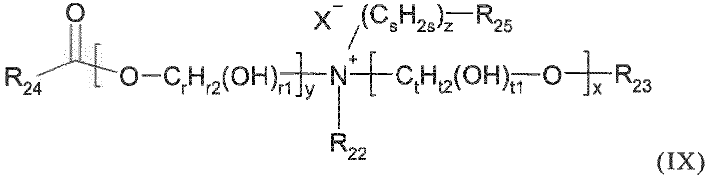 in which:
R₂₂ is chosen from linear or branched C₁-C₆ alkyl radicals and C₁-C₆ hydroxyalkyl or dihydroxyalkyl radicals;
R₂₃ is chosen from:
- the group 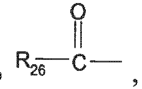
- radicals R₂₇, which are linear or branched, saturated or unsaturated C₁-C₂₂ hydrocarbon-based radicals,
- a hydrogen atom,
R₂₅ is chosen from:
- the group 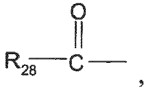
- radicals R₂₉, which are linear or branched, saturated or unsaturated C₁-C₆ hydrocarbon-based radicals,
- a hydrogen atom,
R₂₄, R₂₆ and R₂₈, which may be identical or different, are chosen from linear or branched, saturated or unsaturated C₇-C₂₁ hydrocarbon-based radicals;
r, s and t, which may be identical or different, are integers ranging from 2 to 6;
y is an integer ranging from 1 to 10;
x and z, which may be identical or different, are integers ranging from 0 to 10;
r₁ or t₁ = 0 or 1, r₂ + r₁ = 2r and t₂ + t₁ = 2t,
X⁻ is a simple or complex, organic or mineral anion;
with the proviso that the sum x + y + z is from 1 to 15, that when x is 0, then R₂₃ denotes R₂₇ and that when z is 0, then R₂₅ denotes R₂₉.

6. Composition according to any one of the preceding claims, **characterized in that** the cationic surfactant(s) are chosen from behenyltrimethylammonium, distearyldimethylammonium, cetyltrimethylammonium and benzyldimethylstearylammonium chlorides, or else from palmitylamidopropyltrimethylammonium or stearamidopropyldimethyl(myristyl acetate)-ammonium chlorides.

7. Composition according to any of the preceding claims, **characterized in that** the associative polymer is a cationic amphiphilic associative polymer chosen from quaternized hydroxyethylcelluloses modified with at least one alkyl group having 10 to 22 carbon atoms.

8. Composition according to anyone of the preceding claims, **characterized in that** the thickening agents different from (iii) are non ionic preferably chosen from fatty alcohols, fatty acid amides, oxyalkylenated fatty acid esters and nonionic thickening polymers, or mixtures thereof.

9. Composition according to anyone of the preceding claims, **characterized in that** the thickening agents are:
- fatty alcohols chosen from lauryl alcohol, cetyl alcohol, stearyl alcohol, oleyl alcohol, behenyl alcohol, linoleyl alcohol, undecylenyl alcohol, palmitoleyl alcohol, arachidonyl alcohol and erucyl alcohol, and mixtures thereof,
- fatty acid amides chosen from amides of a C₂-C₁₀ alkanolamine and of a C₁₄-C₃₀ fatty acid, and even more preferentially from amides of a C₂-C₁₀ alkanolamine and of a C₁₄-C₂₂ fatty acid,
- oxyalkylenated derivatives of fatty acid esters chosen from the compounds of formula (A) below:
R₁-CO(X)ₙ-(OCH₂CH₂)ₘ-O-(CO)ₚ-R₂ (A)
X denoting a linear or branched C₁-C₄ alkylene radical, and preferably the radical having the following formula: 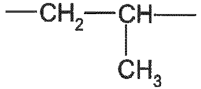
n denoting 0 or 1,
p denoting 0 or 1,
m ranging from 50 to 200, and
R₁ denoting a linear or branched C₉-C₂₉ alkyl or alkenyl radical and R₂ denoting a hydrogen atom or a linear or branched C₉-C₂₉ alkyl or alkenyl radical; and
- non ionic non associative polymers chosen from:
(i) nonionic homopolymers and copolymers containing ethylenically unsaturated monomers of ester and/or amide type,
(ii) vinylpyrrolidone homopolymers or copolymers,
(iii) polysaccharides.

10. Process for treating the hair, comprising the application to the said hair of the composition according to any of the preceding claims.

11. Process according claim 10, **characterized in that** the application of the composition is being followed by drying hair at room temperature or at a temperature above 40°C.

12. Use of the composition according to anyone of claims 1 to 9 as a hair conditioner, preferably used after the application of a shampoo.

## Patentansprüche

1. Zusammensetzung, umfassend:
(i) ein oder mehrere Aminosilikone,
(ii) ein oder mehrere kationische Tenside, die von (i) verschieden sind,
(iii) ein oder mehrere assoziative Polymere und
(iv) ein oder mehrere Verdickungsmittel, die von den assoziativen Polymeren (iii) verschieden sind,
wobei es sich bei den assoziativen Polymeren um kationische amphiphile assoziative Polymere handelt, die aus quaternisierten Hydroxyethylcellulosen, die mit mindestens einer Alkylgruppe mit mindestens 8 Kohlenstoffatomen modifiziert sind, ausgewählt sind,
wobei die assoziativen Polymere in der Zusammensetzung in einer Menge von 0,01 Gew.-% bis 5 Gew.-%, bezogen auf das Gesamtgewicht der Zusammensetzung, vorliegen.

2. Zusammensetzung nach Anspruch 1, **dadurch gekennzeichnet, dass** die Aminosilikone ausgewählt sind aus :
(a) den Verbindungen, die der nachstehenden Formel (I) entsprechen:
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
wobei:
T für ein Wasserstoffatom oder einen Phenyl-, Hydroxyl(-OH)- oder C₁-C₈-Alkylrest und vorzugsweise Methyl oder ein C₁-C₈-Alkoxy, vorzugsweise Methoxy, steht,
a für die Zahl 0 oder eine ganze Zahl von 1 bis 3 und vorzugsweise 0 steht,
b für 0 oder 1 und insbesondere 1 steht,
m und n für solche Zahlen stehen, dass die Summe (n + m) von 50 bis 150 variiert, wobei n eine Zahl von 49 bis 149 bedeutet und m eine Zahl von 1 bis 10 bedeutet;
R¹ für einen einwertigen Rest der Formel -C_{q}H_{2q}L steht, wobei q für eine Zahl von 2 bis 8 steht und
L für eine gegebenenfalls quaternisierte Aminogruppe steht, die aus den folgenden Gruppen ausgewählt ist:
- N(R²)-CH₂-CH₂-N(R²)₂;
- N(R²)₂; -N⁺(R²)₃Q⁻, wie -N⁺(R²)(H)₂Q⁻, -N⁺(R²)₂HQ⁻;
- N(R²)-CH₂-CH₂-N⁺(R²)(H)₂Q⁻;
wobei R² ein Wasserstoffatom, einen Phenylrest, einen Benzylrest oder einen gesättigten einwertigen Rest auf Kohlenwasserstoffbasis, beispielsweise einen C₁-C₂₀-Alkylrest, bedeutet und
Q⁻ für ein Halogenidion, beispielsweise Fluorid, Chlorid, Bromid oder Iodid, steht,
(b) den Verbindungen, die der nachstehenden Formel (II) entsprechen: 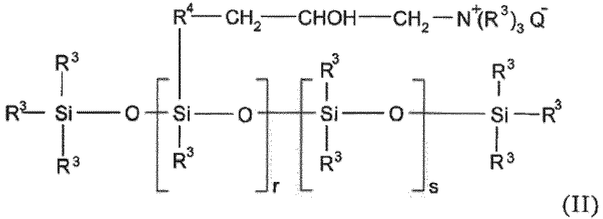 wobei:
R³ für einen einwertigen C₁-C₁₈-Rest auf Kohlenwasserstoffbasis und insbesondere einen C₁-C₁₈-Alkyl- oder C₂-C₁₈-Alkenylrest, beispielsweise Methyl, steht;
R⁴ für einen zweiwertigen Rest auf Kohlenwasserstoffbasis, insbesondere einen C₁-C₁₈-Alkylenrest oder einen zweiwertigen C₁-C₁₈-Alkylenoxyrest und beispielsweise einen C₁-C₈-Alkylenoxyrest steht;
Q⁻ für ein Halogenidion, insbesondere Chlorid, steht;
r für einen mittleren statistischen Wert von 2 bis 20 und insbesondere von 2 bis 8 steht;
s für einen mittleren statistischen Wert von 20 bis 200 und insbesondere von 20 bis 50 steht;
(c) den quartären Ammoniumsilikonen der Formel (III) : 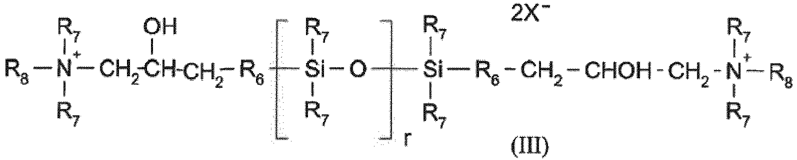 wobei:
die Reste R₇, die gleich oder verschieden sein können, für einen einwertigen Rest auf Kohlenwasserstoffbasis mit 1 bis 18 Kohlenstoffatomen und insbesondere einen C₁-C₁₈-Alkylrest, einen C₂-C₁₈-Alkenylrest oder einen Ring mit 5 oder 6 Kohlenstoffatomen, beispielsweise Methyl, stehen;
R₆ für einen zweiwertigen Rest auf Kohlenwasserstoffbasis, insbesondere einen C₁-C₁₈-Alkylenrest oder einen zweiwertigen C₁-C₁₈-Alkylenoxyrest, beispielsweise einen C₁-C₈-Alkylenoxyrest, der über eine SiC-Bindung an das Si-Atom gebunden ist, steht;
die Reste R₈, die gleich oder verschieden sein können, für ein Wasserstoffatom, einen einwertigen Rest auf Kohlenwasserstoffbasis mit 1 bis 18 Kohlenstoffatomen und insbesondere einen C₁-C₁₈-Alkylrest, einen C₂-C₁₈-Alkenylrest oder einen Rest -R₆-NHCOR₇ stehen;
X⁻ für ein Anion wie ein Halogenidion, insbesondere Chlorid, oder ein Salz einer organischen Säure (Acetat usw.) steht;
r für einen mittleren statistischen Wert von 2 bis 200 und insbesondere von 5 bis 100 steht;
(d) den Aminosilikonen der nachstehenden Formel (IV) : 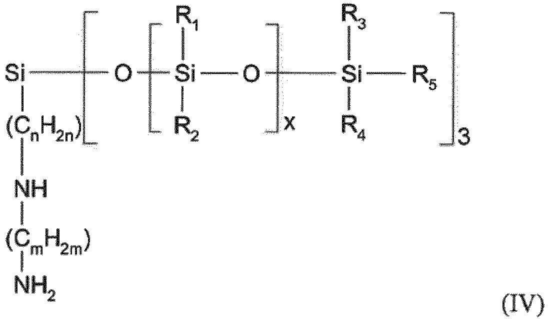 wobei:
- R₁, R₂, R₃ und R₄, die gleich oder verschieden sein können, für einen C₁-C₄-Alkylrest oder eine Phenylgruppe stehen,
- R₅ für einen C₁-C₄-Alkylrest oder eine Hydroxylgruppe steht,
- n für eine ganze Zahl im Bereich von 1 bis 5 steht,
- m für eine ganze Zahl im Bereich von 1 bis 5 steht und
wobei x so gewählt ist, dass die Aminzahl zwischen 0,01 und 1 meq/g liegt;
(e) polyoxyalkylenierten Aminosilikonen mit mehreren Blöcken des Typs (AB)n, wobei A für einen Polysiloxanblock steht und B für einen Polyoxyalkylenblock steht, mit mindestens einer Amingruppe.

3. Zusammensetzung nach Anspruch 1 oder 2, **dadurch gekennzeichnet, dass** die Aminosilikone ausgewählt sind aus polyoxyalkylenierten Aminosilikonen mit mehreren Blöcken des Typs (AB)n, wobei A für einen Polysiloxanblock steht und B für einen Polyoxyalkylenblock steht, mit mindestens einer Amingruppe, wobei die Silikone vorzugsweise aus Wiederholungseinheiten mit den folgenden allgemeinen Formeln bestehen:
[-(SiMe₂O)ₓSiMe₂-R-N(R'')-R'-O-(C₂H₄O)ₐ(C₃H₆O)_{b}-R'-N(H)-R-]
oder alternativ dazu
[-(SiMe₂O)ₓSiMe₂-R-N(R'')-R'-O-(C₂H₄O)ₐ(C₃H₆O)_{b}-],
wobei:
- a für eine ganze Zahl größer oder gleich 1, vorzugsweise im Bereich von 5 bis 200 und spezieller im Bereich von 10 bis 100, steht;
- b für eine ganze Zahl zwischen 0 und 200, vorzugsweise im Bereich von 4 bis 100 und spezieller im Bereich von 5 bis 30, steht;
- x für eine ganze Zahl im Bereich von 1 bis 10.000 und spezieller von 10 bis 5000 steht;
- R" für ein Wasserstoffatom oder ein Methyl steht;
- die Reste R, die gleich oder verschieden sein können, für einen linearen oder verzweigten zweiwertigen C₂-C₁₂-Rest auf Kohlenwasserstoffbasis, der gegebenenfalls ein oder mehrere Heteroatome wie Sauerstoff umfasst, stehen; R bevorzugt für einen Ethylenrest, einen linearen oder verzweigten Propylenrest, einen linearen oder verzweigten Butylenrest oder einen
- CH₂CH₂CH₂OCH(OH)CH₂-Rest steht; R vorzugsweise für einen -CH₂CH₂CH₂OCH(OH)CH₂-Rest steht;
- die Reste R', die gleich oder verschieden sein können, für einen linearen oder verzweigten zweiwertigen C₂-C₁₂-Rest auf Kohlenwasserstoffbasis, der gegebenenfalls ein oder mehrere Heteroatome wie Sauerstoff umfasst, stehen; R' bevorzugt für einen Ethylenrest, einen linearen oder verzweigten Propylenrest, einen linearen oder verzweigten Butylenrest oder einen -CH₂CH₂CH₂OCH(OH)CH₂-Rest steht; R' vorzugsweise für einen -CH(CH₃)-CH2-Rest steht.

4. Zusammensetzung nach einem der vorhergehenden Ansprüche, **dadurch gekennzeichnet, dass** das kationische Tensid bzw. die kationischen Tenside aus Salzen von gegebenenfalls polyoxyalkylenierten primären, sekundären oder tertiären Fettaminen und quaternären Ammoniumsalzen und Mischungen davon ausgewählt sind.

5. Zusammensetzung nach einem der vorhergehenden Ansprüche, **dadurch gekennzeichnet, dass** es sich bei dem kationischen Tensid um ein quaternäres Ammoniumsalz handelt, das ausgewählt ist aus:
- denjenigen der folgenden allgemeinen Formel (VI) : 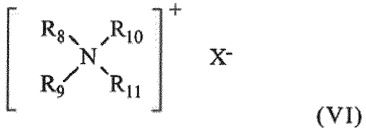 wobei die Reste R₈ bis R₁₁, die gleich oder verschieden sein können, für einen linearen oder verzweigten aliphatischen Rest mit 1 bis 30 Kohlenstoffatomen oder einen aromatischen Rest wie Aryl oder Alkylaryl stehen; X für ein Anion aus der Gruppe der Halogenide, Phosphate, Acetate, Lactate, (C₂-C₆)Alkylsulfate, Alkyl- oder Alkylarylsulfonate steht;
- quaternären Ammoniumsalzen von Imidazolin;
- di- oder triquaternären Ammoniumsalzen, insbesondere der Formel (VIII): 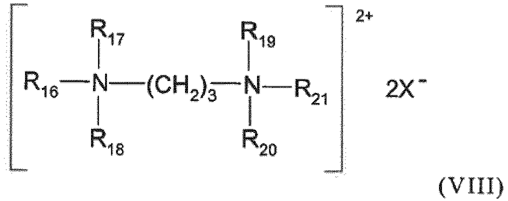 wobei R₁₆ für einen Alkylrest mit ungefähr 16 bis 30 Kohlenstoffatomen steht, der gegebenenfalls hydroxyliert und/oder durch ein oder mehrere Sauerstoffatome unterbrochen und gegebenenfalls verzweigt ist, R₁₇ aus einem Alkylrest mit 1 bis 4 Kohlenstoffatomen oder einer Gruppe (R₁₆ₐ)(R₁₇ₐ)(R₁₈ₐ)N⁺-(CH₂)₃- ausgewählt ist, R₁₆ₐ, R₁₇ₐ, R₁₈ₐ, R₁₈, R₁₉, R₂₀ und R₂₁, die gleich oder verschieden sein können, aus Wasserstoff und einem Alkylrest mit 1 bis 4 Kohlenstoffatomen ausgewählt sind und X⁻ für ein Anion aus der Gruppe der Halogenide, Acetate, Phosphate, Nitrate und Methylsulfate steht;
- quaternären Ammoniumsalzen mit mindestens einer Esterfunktion der nachstehenden Formel (IX): 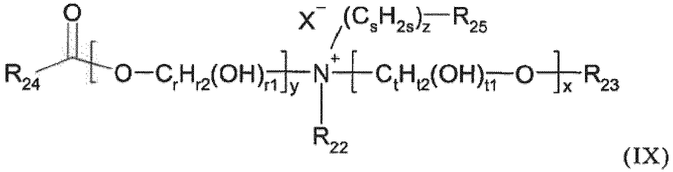 wobei:
R₂₂ aus linearen oder verzweigten C₁-C₆-Alkylresten und C₁-C₆-Hydroxyalkyl- oder -Dihydroxyalkylresten ausgewählt ist;
R₂₃ aus:
- der Gruppe 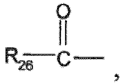
- Resten R₂₇, bei denen es sich um lineare oder verzweigte, gesättigte oder ungesättigte C₁-C₂₂-Reste auf Kohlenwasserstoffbasis handelt,
- einem Wasserstoffatom
ausgewählt ist,
R₂₅ aus :
- der Gruppe 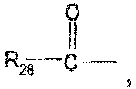
- Resten R₂₉, bei denen es sich um lineare oder verzweigte, gesättigte oder ungesättigte C₁-C₆-Reste auf Kohlenwasserstoffbasis handelt,
- einem Wasserstoffatom
ausgewählt ist,
R₂₄, R₂₆ und R₂₈, die gleich oder verschieden sein können, aus linearen oder verzweigten, gesättigten oder ungesättigten C₇-C₂₁-Resten auf Kohlenwasserstoffbasis ausgewählt sind;
r, s und t, die gleich oder verschieden sein können, für ganze Zahlen im Bereich von 2 bis 6 stehen;
y für eine ganze Zahl im Bereich von 1 bis 10 steht;
x und z, die gleich oder verschieden sein können, für ganze Zahlen im Bereich von 0 bis 10 stehen;
r₁ oder t₁ = 0 oder 1, r₂ + r₁ = 2r und t₂ + t₁ = 2t,
X⁻ für ein einfaches oder komplexes, organisches oder mineralisches Anion steht;
mit der Maßgabe, dass die Summe x + y + z 1 bis 15 beträgt, dass dann, wenn x für 0 steht, R₂₃ für R₂₇ steht und dann, wenn z für 0 steht, R₂₅ für R₂₉ steht.

6. Zusammensetzung nach einem der vorhergehenden Ansprüche, **dadurch gekennzeichnet, dass** das kationische Tensid bzw. die kationischen Tenside aus Behenyltrimethylammoniumchlorid, Distearyldimethylammoniumchlorid, Cetyltrimethylammoniumchlorid und Benzyldimethylstearylammoniumchlorid oder auch Palmitylamidopropyltrimethylammoniumchlorid oder Stearamidopropyldimethyl(myristyl-acetat)ammoniumchlorid ausgewählt ist bzw. sind.

7. Zusammensetzung nach einem der vorhergehenden Ansprüche, **dadurch gekennzeichnet, dass** es sich bei dem assoziativen Polymer um ein kationisches amphiphiles und assoziatives Polymer handelt, das aus quaternisierten Hydroxyethylcellulosen, die mit mindestens einer Alkylgruppe mit 10 bis 22 Kohlenstoffatomen modifiziert sind, ausgewählt ist.

8. Zusammensetzung nach einem der vorhergehenden Ansprüche, **dadurch gekennzeichnet, dass** die Verdickungsmittel, die von (iii) verschieden sind, nichtionisch sind und vorzugsweise aus Fettalkoholen, Fettsäureamiden, oxyalkylierten Fettsäureestern und nichtionischen verdickenden Polymeren oder Mischungen davon ausgewählt sind.

9. Zusammensetzung nach einem der vorhergehenden Ansprüche, **dadurch gekennzeichnet, dass** es sich bei den Verdickungsmitteln um Folgendes handelt:
- Fettalkohole, ausgewählt aus Laurylalkohol, Cetylalkohol, Stearylalkohol, Oleylalkohol, Behenylalkohol, Linoleylalkohol, Undecylenylalkohol, Palmitoleylalkohol, Arachidonylalkohol und Erucylalkohol und Mischungen davon,
- Fettsäureamide, ausgewählt aus Amiden eines C₂-C₁₀-Alkanolamins und einer C₁₄-C₃₀-Fettsäure und noch weiter bevorzugt aus Amiden eines C₂-C₁₀-Alkanolamins und einer C₁₄-C₂₂-Fettsäure,
- oxyalkylenierte Derivate von Fettsäureestern, ausgewählt aus den Verbindungen der nachstehenden Formel (A):
R₁-CO(X)ₙ-(OCH₂CH₂)ₘ-O-(CO)ₚ-R₂ (A)
wobei X für einen linearen oder verzweigten C₁-C₄-Alkylenrest und vorzugsweise den Rest der folgenden Formel steht: 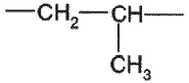
wobei n für 0 oder 1 steht,
wobei p für 0 oder 1 steht,
wobei m im Bereich von 50 bis 200 liegt und
wobei R₁ für einen linearen oder verzweigten C₉-C₂₉-Alkyl- oder -Alkenylrest steht und wobei R² für ein Wasserstoffatom oder einen linearen oder verzweigten C₉-C₂₉-Alkyl- oder -Alkenylrest steht; und
- nichtionische nichtassoziative Polymere, ausgewählt aus:
(i) nichtionischen Homopolymeren und Copolymeren, die ethylenisch ungesättigte Monomere von Ester- und/oder Amid-Typ enthalten,
(ii) Vinylpyrrolidon-Homopolymeren oder -Copolymeren,
(iii) Polysacchariden.

10. Verfahren zur Behandlung des Haars, bei dem man die Zusammensetzung nach einem der vorhergehenden Ansprüche auf das Haar aufbringt.

11. Verfahren nach Anspruch 10, **dadurch gekennzeichnet, dass** man nach dem Aufbringen der Zusammensetzung das Haar bei Raumtemperatur oder bei einer Temperatur über 40 °C trocknet.

12. Verwendung der Zusammensetzung nach einem der Ansprüche 1 bis 9 als Haarconditioner, der vorzugsweise nach dem Aufbringen eines Shampoos verwendet wird.

## Revendications

1. Composition comprenant :
i) une ou plusieurs silicones aminées,
ii) un ou plusieurs tensioactifs cationiques, différents du ou des composés (i),
iii) un ou plusieurs polymères associatifs,
iv) et un ou plusieurs agents épaississants, différents du ou des polymères associatifs (iii),
étant entendu que les polymères associatifs sont des polymères associatifs cationiques et amphiphiles, choisis parmi les hydroxyéthyl-celluloses quaternisées et modifiées avec au moins un groupe alkyle comportant au moins 8 atomes de carbone,
et que les polymères associatifs sont présents dans ladite composition en une proportion de 0,01 à 5 %, en poids rapporté au poids total de la composition.

2. Composition selon la revendication 1, **caractérisée en ce que** les silicones aminées sont choisies parmi les composés suivants :
a) les composés de formule (I) ci-dessous :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
dans laquelle
- T représente un atome d'hydrogène ou un groupe phényle, hydroxyle (-OH), alkyle en C₁-C₈, de préférence méthyle, ou alcoxy en C₁-C₈, de préférence méthoxy,
- l'indice a vaut 0 ou est un nombre entier valant de 1 à 3, et vaut de préférence 0
- l'indice b vaut 0 ou 1, en particulier 1,
- les indices m et n sont des nombres tels que leur somme n+m vaut de 50 à 150, étant entendu que n est un nombre valant de 49 à 149 et que m est un nombre valant de 1 à 10,
- et R¹ représente un groupe monovalent de formule -C_{q}H_{2q}L dans laquelle l'indice q est un nombre valant de 2 à 8 et L représente un groupe amino, éventuellement quaternisé, choisi parmi les groupes de formules suivantes :
- N(R²)-CH₂-CH₂-N(R²)₂;
- N(R²)₂;
- N⁺(R²)₃Q⁻, telles -N⁺(R²)(H)₂Q⁻, -N⁺(R²)₂(H)Q⁻ ;
- N(R²)-CH₂-CH₂-N⁺(R²)(H)₂Q⁻,
dans lesquelles R² représente un atome d'hydrogène, un groupe phényle ou benzyle, ou un groupe hydrocarboné saturé monovalent, par exemple alkyle en C₁-C₂₀, et Q⁻ représente un ion halogénure, par exemple fluorure, chlorure, bromure ou iodure ;
b) les composés de formule (II) ci-dessous : 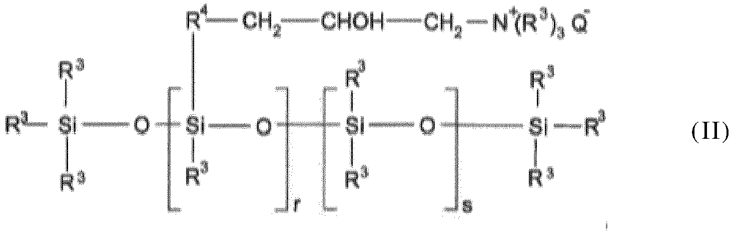 dans laquelle
- R³ représente un groupe hydrocarboné monovalent en C₁-C₁₈, en particulier alkyle en C₁-C₁₈ ou alcényle en C₂-C₁₈,
par exemple méthyle,
- R⁴ représente un groupe hydrocarboné divalent, en particulier alkylène en C₁-C₁₈, ou un groupe divalent alkylène-oxy en C₁-C₁₈, par exemple en C₁-C₈,
- Q⁻ représente un ion halogénure, en particulier chlorure,
- l'indice r représente une moyenne statistique valant de 2 à 20 et en particulier de 2 à 8,
- et l'indice s représente une moyenne statistique valant de 20 à 200 et en particulier de 20 à 50 ;
c) les composés de type silicone-ammonium quaternaire, de formule (III) ci-dessous : 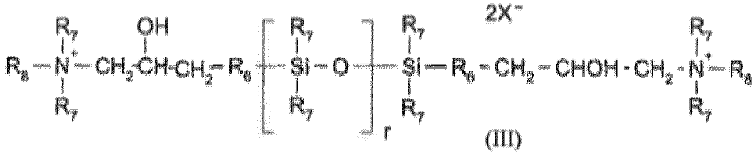 dans laquelle
- Les radicaux R₇, qui peuvent être identiques ou différents, représentent des groupes hydrocarbonés monovalents comportant de 1 à 18 atomes de carbone, en particulier des groupes alkyles en C₁-C₁₈, alcényle en C₂-C₁₈, ou cycliques comportant 5 ou 6 atomes de carbone, et par exemple des groupes méthyle,
- R₆ représente un groupe hydrocarboné divalent, en particulier alkylène en C₁-C₁₈, ou un groupe divalent alkylène-oxy en C₁-C₁₈, par exemple en C₁-C₈, lié à l'atome de silicium par une liaison Si-C,
- Les radicaux R₈, qui peuvent être identiques ou différents, représentent un atome d'hydrogène, un groupe hydrocarboné monovalent comportant de 1 à 18 atomes de carbone, en particulier alkyle en C₁-C₁₈ ou alcényle en C₂-C₁₈, ou un groupe -R₆-NHCOR₇,
- X⁻ représente un anion, comme un ion halogénure, en particulier chlorure, ou un anion d'un sel d'acide organique (acétate, etc.),
- et l'indice r représente une moyenne statistique valant de 2 à 200 et en particulier de 5 à 100 ;
d) les silicones aminées de formule (IV) ci-dessous : 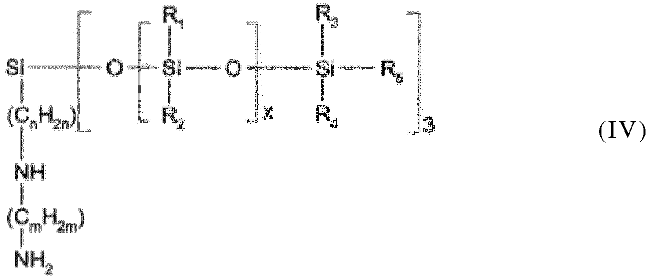 dans laquelle
- les radicaux R₁, R₂, R₃ et R₄, qui peuvent être identiques ou différentes, représentent un groupe alkyle en C₁-C₄ ou un groupe phényle,
- R₅ représente un groupe alkyle en C₁-C₄ ou un groupe hydroxyle,
- l'indice n est un nombre entier valant de 1 à 5,
- l'indice m est un nombre entier valant de 1 à 5,
- et l'indice x est un nombre choisi de telle sorte que l'indice d'amine vaille entre 0,01 et 1 méq/g ;
e) et les silicones aminées polyoxyalkylénées multi-blocs, symbolisées par (AB)n où A représente un bloc polysiloxane et B représente un bloc poly(oxy-alkylène) comportant au moins un groupe amino.

3. Composition selon les revendications 1 ou 2, **caractérisée en ce que** les silicones aminées sont choisies parmi les silicones aminées polyoxyalkylénées multi-blocs, symbolisées par (AB)n où A repré-sente un bloc polysiloxane et B représente un bloc poly(oxy-alkylène) comportant au moins un groupe amino, lesquelles silicones sont de préférence constituées de motifs répétés présentant les formules générales suivantes :
[-(SiMe₂O)ₓSiMe₂-R-N(R")-R'-O(C₂H_{4O})ₐ(C₃H₆O)_{b}-R'-N(H)-R-]
ou bien encore
[-(SiMe₂O)ₓSiMe₂-R-N(R")-R'-O(C₂H₄O)ₐ(C₃H₆O)_{b}-]
dans lesquelles formules
- l'indice a est un nombre entier supérieur ou égal à 1, valant de préférence de 5 à 200 et mieux encore de 10 à 100,
- l'indice b est un nombre entier valant de 0 à 200, de préférence de 4 à 100 et mieux encore de 5 à 30,
- l'indice x est un nombre entier valant de 1 à 10 000 et en particulier de 10 à 5 000,
- R" représente un atome d'hydrogène ou un groupe méthyle,
- les radicaux R, qui peuvent être identiques ou différentes, représentent un groupe hydrocarboné divalent en C₂-C₁₂, linéaire ou ramifié, et comportant, éventuellement, un ou plusieurs hétéroatomes, comme un ou des atomes d'oxygène, étant entendu que, de préférence, R représente un groupe éthylène, propylène linéaire ou ramifié, ou butylène linéaire ou ramifié, ou le groupe -CH₂CH₂CH₂OCH(OH)CH₂-, préférentiellement R représente le groupe -CH₂CH₂CH₂OCH(OH)CH₂-,
- les radicaux R', qui peuvent être identiques ou différentes, représentent un groupe hydrocarboné divalent en C₂-C₁₂, linéaire ou ramifié, et comportant, éventuellement, un ou plusieurs hétéroatomes, comme un ou des atomes d'oxygène, étant entendu que, de préférence, R' représente un groupe éthylène, propylène linéaire ou ramifié, ou butylène linéaire ou ramifié, ou le groupe -CH₂CH₂CH₂OCH(OH)CH₂-, et plus préférentiellement R' représente le groupe -CH(CH₃)CH₂-.

4. Composition selon l'une quelconque des revendications précédentes, **caractérisée en ce que** le ou les tensioactifs cationiques sont choisis parmi les sels d'amines grasses primaires, secondaires ou tertiaires, éventuellement polyoxyalkoxylées, et les sels d'ammonium quaternaire, ainsi que leurs mélanges.

5. Composition selon l'une quelconque des revendications précédentes, **caractérisée en ce que** le tensioactif cationique est un sel d'ammonium quaternaire choisi parmi :
- ceux de formule générale (VI) ci-dessous : 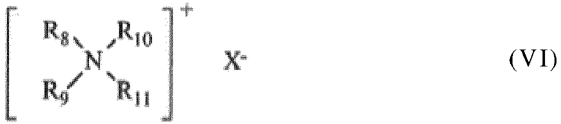 dans laquelle
- les radicaux R₈ à R₁₁, qui peuvent être identiques ou différents, représentent un groupe aliphatique linéaire ou ramifié, comportant de 1 à 30 atomes de carbone, ou un groupe aromatique comme un groupe aryle ou alkyl-aryle,
- et X⁻ représente un anion choisi dans l'ensemble formé par les ions halogénures, phosphates, acétate, lactate, (alkyle en C₂-C₆)-sulfates, alkyl-sulfonates et alkyl-aryl-sulfonates,
- les sels d'ammonium quaternaire d'imidazoline,
- les sels de diammonium ou triammonium quaternaire, en particulier ceux de formule (VIII) ci-dessous : 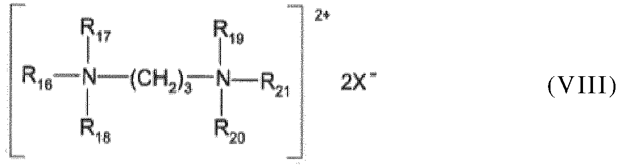 dans laquelle
- R₁₆ représente un groupe alkyle comportant environ 16 à 30 atomes de carbone, qui est éventuellement hydroxylé et/ou interrompu avec un ou plusieurs atomes d'oxygène, et éventuellement ramifié,
- R₁₇ représente un groupe alkyle comportant de 1 à 4 atomes de carbone, ou un groupe (R₁₆ₐ)(R₁₇ₐ)(R₁₈ₐ)N⁺-(CH₂)₃-,
- les radicaux R₁₆ₐ, R₁₇ₐ, R₁₈ₐ, R₁₈, R₁₉, R₂₀ et R₂₁, qui peuvent être identiques ou différents, représentent un atome d'hydrogène, un groupe alkyle comportant de 1 à 4 atomes de carbone,
- et X⁻ représente un anion choisi dans l'ensemble formé par les ions halogénures, acétate, phosphates, nitrate et méthyl-sulfate ;
- les sels d'ammonium quaternaire comportant au moins un groupe fonctionnel ester, de formule (IX) ci-dessous : 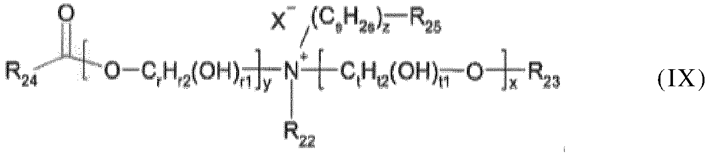 dans laquelle
- R₂₂ représente un radical choisi parmi les groupes alkyles en C₁-C₆ linéaires ou ramifiés, les groupes hydroxy-alkyle ou dihydroxy-alkyle en C₁-C₆,
- R₂₃ représente un radical choisi parmi les groupes 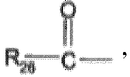 les radicaux R₂₇ qui sont des groupes hydrocarbonés en C₁-C₂₂, linéaires ou ramifiés, saturés ou insaturés, un atome d'hydrogène,
- R₂₅ est choisi parmi le groupe 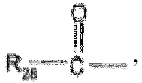 les radicaux R₂₉ représentant des groupes hydrocarbonés en C₁-C₆, linéaires ou ramifiés, saturés ou insaturés, et un atome d'hydrogène,
- les radicaux R₂₄, R₂₆ et R₂₈, identiques ou différents, sont choisis parmi les groupes hydrocarbonés en C₇-C₂₁, linéaires ou ramifiés, saturés ou insaturés ;
- les indices r, s et t, identiques ou différents, sont des nombres entiers entre 2 et 6,
- l'indice y est un nombre entier valant de 1 à 10,
- les indices x et z sont des nombres entiers, identiques ou différents, entre 0 et 10,
- l'indice r₁ ou l'indice t₁ vaut 0 ou 1, avec r₂ + r₁ = 2r et t₂ + t₁ = 2t,
- et X⁻ représente un anion organique ou minéral, simple ou complexe,
sous réserve que la somme x + y + z est comprise entre 1 à 15, que si x vaut 0, alors R₂₃ représente un radical R₂₇, et que si z vaut 0, alors R₂₅ représente un radical R₂₉.

6. Composition selon l'une quelconque des revendications précédentes, **caractérisée en ce que** le ou les tensioactif(s) cationique(s) est ou sont choisi(s) parmi les chlorures de béhényltriméthylammonium, de distéaryldiméthylammonium, de cétyltriméthylammonium et de benzylstéaryldiméthylammonium, ou encore parmi les chlorures de palmitylamidopropyltriméthylammonium et de stéaramidopropyldiméthyl(myristyl acétate) ammonium.

7. Composition selon l'une quelconque des revendications précédentes, **caractérisée en ce que** le polymère associatif est un polymère associatif cationique amphiphile choisi parmi les hydroxyéthylcelluloses quaternisées modifiées avec au moins un groupe alkyle comportant de 10 à 22 atomes de carbone

8. Composition selon l'une quelconque des revendications précédentes, **caractérisée en ce que** les agents épaississants différents des polymères (iii) sont non-ioniques et choisis de préférence parmi les alcools gras, les amides d'acide gras, les esters d'acide gras oxyalkylénés et les polymères épaississants non-ioniques, ou les mélanges de tels composés.

9. Composition selon l'une quelconque des revendications précédentes, **caractérisée en ce que** les agents épaississants sont
- des alcools gras choisis parmi les alcools laurylique, cétylique, stéarylique, oléylique, béhénylique, linoléylique, undécylénylique, palmitoléylique, arachidonylique et érucylique, ainsi que leurs mélanges,
- des amides d'acide gras choisis parmi les amides dérivés d'une alcanolamine en C₂-C₁₀ et d'un acide gras en C₁₄-C₃₀, et de préférence, parmi les amides dérivés d'une alcanolamine en C₂-C₁₀ et d'un acide gras en C₁₄-C₂₂,
- des dérivés oxyalkylénés d'esters d'acide gras, choisis parmi les composés de formule (A) ci-dessous :
R-CO(X)ₐ-(OCH₂CH₂)ₘ-O-(CO)ₚ-R2 (A)
dans laquelle
- X représente un groupe alkylène en C₁-C₄ linéaire ou ramifié, et de préférence le groupe de formule suivante : 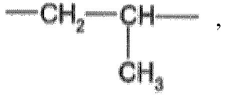
- l'indice n vaut 0 ou 1,
- l'indice p vaut 0 ou 1,
- l'indice m vaut de 50 à 200,
- R₁ représente un groupe alkyle ou alkylène en C₉-C₂₉, linéaire ou ramifié,
- et R₂ représente un atome d'hydrogène ou un groupe alkyle ou alkylène en C₉-C₂₉, linéaire ou ramifié,
- et des polymères non-ioniques non-associatifs, choisis parmi
i) les homopolymères et copolymères non-ioniques comportant des monomères à insaturation éthylénique de type ester et/ou de type amide,
ii) les homopolymères et copolymères de vinylpyrrolidone,
iii) les polysaccharides.

10. Procédé de traitement de cheveux, comportant l'application, sur lesdits cheveux, d'une composition selon l'une quelconque des revendications précédentes.

11. Procédé selon la revendication 10, **caractérisé en ce que** l'application de la composition est suivie d'un séchage des cheveux à température ambiante ou à une température supérieure à 40 °C.

12. Utilisation d'une composition selon l'une quelconque des revendications 1 à 9 en tant que conditionneur capillaire, utilisé de préférence après l'application d'un shampooing.
